# FASCICULE DE BREVET EUROPEEN

(11) **EP 1 346 750 B1**
(45) Date de publication et mention de la délivrance du brevet: **01.06.2005**
(21) Numéro de dépôt: 03290707.3
(22) Date de dépôt: 20.03.2003
(51) Int. Cl.: A61N 1/368

(54) **Dispositif médical implantable actif tel que stimulateur cardiaque, défibrillateur et/ou cardioverteur, à commutation de mode DDD/AAI perfectionnée**
Aktive implantierbare medizinische Vorrichtung, insbesondere Herzschrittmacher, Defibrillator, und/oder Kardiovertierer, mit verbesserter Betriebsartumschaltung DDD/AAI
Active implantable medical device, in particular pacemaker, defibrillator and/or cardioverter, with improved mode switching DDD/AAI

(30) Priorité: 22.03.2002 FR 0203606
(43) Date de publication de la demande: 24.09.2003
(73) Titulaire: ELA MEDICAL (Société anonyme), 92541 Montrouge (FR)
(72) Inventeur: Amblard, Amel, 92290 Chatenay Malabry (FR); Limousin, Marcel, 75014 Paris (FR)
(74) Mandataire: Dupuis-Latour, Dominique

(56) Documents cités:
- EP-A- 0 488 904
- WO-A-01/70104
- US-A- 5 893 882
- US-A- 5 928 271

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite" (triple ou quadruple chambre), défibrillateurs et/ou cardioverteurs, permettant de délivrer au coeur des impulsions électriques de faible énergie pour le traitement des troubles du rythme cardiaque.

Elle concerne plus particulièrement ceux de ces dispositifs qui comprennent des circuits de stimulation et de détection à la fois sur l'oreillette et sur le ventricule et peuvent opérer selon deux modes de fonctionnement, DDD ou AAI (le mode AAI étant en fait un mode DDD comprenant un délai atrio-ventriculaire allongé). Ces dispositifs peuvent être pourvus d'un mode dénommé "DDD-CAM" assurant une commutation automatique du mode (CAM) de DDD en AAI et inversement.

Le mode de fonctionnement de base d'un stimulateur DDD/AAI est un mode AAI, avec une stimulation auriculaire simple chambre et une surveillance (détection) de l'activité ventriculaire. Ce mode est maintenu tant que la conduction atrio-ventriculaire est normale, c'est-à-dire tant que chaque événement auriculaire (détection auriculaire, correspondant à une activité spontanée, ou stimulation auriculaire) est suivi d'une détection ventriculaire associée.

Dans certaines circonstances peuvent cependant apparaître des blocs atrio-ventriculaires (BAV), dits "paroxystiques", entraînant un défaut temporaire de dépolarisation du ventricule. Dans ce cas, le stimulateur bascule automatiquement en mode DDD automatique, avec des paramètres optimisés pour cette situation de BAV temporaire. Après disparition du BAV, et donc rétablissement de la conduction atrio-ventriculaire spontanée, dès lors qu'un certain nombre de conditions sont remplies le stimulateur retourne automatiquement au mode AAI.

Cette commutation des modes DDD et AAI est par exemple décrit dans le EP-A-0 488 904 (ELA Médical).

Ces dispositifs peuvent être notamment implantés chez des patients souffrant de dysfonctionnements sinusaux susceptibles de produire des troubles du rythme auriculaire.

Le terme "troubles du rythme auriculaire" ou TdRA est un terme générique qui recouvre diverses arythmies auriculaires (épisodes d'accélération non physiologiques du rythme) telles que tachycardie, fibrillation, *flutter,* etc., troubles tous caractérisés, à la détection par un rythme auriculaire rapide. Essentiellement, on considèrera qu'il y a TdRA lorsque le rythme auriculaire détecté dépasse un niveau admissible, ce niveau étant éventuellement fonction d'un degré d'effort évalué par un capteur physiologique.

Pour réduire les épisodes d'arythmie auriculaire, le stimulateur peut être pourvu d'un mode dit "overdrive", qui est un mode particulier assurant une stimulation auriculaire à une fréquence légèrement supérieure au rythme naturel sous-jacent. Ce mode d'overdrive est décrit par exemple dans le EP-A-0 880 979 (ELA Médical).

Le point de départ de l'invention réside dans un certain nombre d'observations effectuées lors d'un suivi clinique de patients appareillés avec des dispositifs susceptibles de mettre en oeuvre les deux fonctionnalités précitées, à savoir DDD-CAM et overdrive.

En effet, le mode de stimulation DDD-CAM est réservé aux patients présentant une conduction atrio-ventriculaire normale. Dans ce mode de stimulation, le stimulateur calcule un DAV de stimulation et de détection en analysant la conduction spontanée du patient. Ce principe de fonctionnement autorise la majorité du temps le maintien d'une activité spontanée ventriculaire. Cependant, ce principe de comportement est limité par une durée maximale du temps de conduction, de l'ordre de 300 ms après détection auriculaire et 350 ms après stimulation auriculaire.

Ce mode de fonctionnement est adapté aux patients présentant une activité sinusale mais, en cas de dysfonctionnement sinusal nécessitant une stimulation auriculaire, il a été constaté que, sur un nombre important de cycles cardiaques :
- soit une stimulation ventriculaire a été déclenchée, c'est-à-dire qu'aucune activité ventriculaire n'a été, ou n'a pu être, détectée,
- soit une fusion est survenue, c'est-à-dire qu'une stimulation est intervenue de façon concomitante à une dépolarisation ventriculaire spontanée, détectée dans la même fenêtre temporelle que la stimulation.

Ces phénomènes sont vraisemblablement dus au fait que la stimulation auriculaire augmente le temps de conduction atrio-ventriculaire, qui excède alors la valeur du DAV programmé, typiquement de 350 ms après stimulation.

Lorsque l'on veut combiner les algorithmes de prévention et de traitement des arythmies auriculaires avec un mode de stimulation DDD-CAM, on constate une augmentation très significative du nombre de cycles avec une stimulation ventriculaire. En effet, l'objet de ces algorithmes est de supprimer l'activité spontanée auriculaire et d'''overdriver" le sinus en permanence.

On sait également que, pour tirer le meilleur parti des algorithmes d'overdrive dans la prévention de la FA, il est important de maintenir une bonne hémodynamique auriculaire. Mais une stimulation ventriculaire prématurée (du fait d'un DAV trop court), empêchant l'expression de la dépolarisation ventriculaire spontanée, modifie la séquence atrio-ventriculaire et fait perdre le bénéfice de l'optimisation auriculo-ventriculaire, l'oreillette n'ayant pas le temps de se remplir (ou de se désemplir) complètement.

L'invention propose de remédier à ces difficultés en apportant un perfectionnement aux dispositifs connus, pour mieux associer prévention et traitement des arythmies auriculaires avec un mode DDD-CAM, par un aménagement approprié de ce mode de fonctionnement.

Plus précisément, l'un des buts de l'invention est de proposer un nouveau mode de commutation permettant, comme dans le cas du mode DDD-CAM connu (par exemple celui décrit par le EP-A-0 880 979 précité), une commutation automatique entre DDD et AAI, mais dans lequel le mode AAI ne sera pas un mode DDD comprenant un délai atrio-ventriculaire allongé.

Essentiellement, il s'agit de faire en sorte que le dispositif reste le plus longtemps possible en mode AAI, en retardant au maximum le basculement en mode DDD et en autorisant une absence d'activité ventriculaire pendant un nombre de cycles prédéterminé, paramétrable.

Le type de dispositif auquel s'applique l'invention est un dispositif médical implantable actif, notamment un stimulateur cardiaque, défibrillateur et/ou cardioverteur, comprenant des moyens de détection d'événements auriculaires et ventriculaires spontanés, des moyens de stimulation ventriculaire et auriculaire, des moyens aptes à faire fonctionner le dispositif en mode DDD, des moyens aptes à faire fonctionner le dispositif en mode AAI avec détection ventriculaire, et des moyens de commutation de mode, aptes à commander le basculement du mode AAI en mode DDD, et inversement, en fonction de critères prédéterminés.

De façon caractéristique de l'invention, les moyens de commutation de mode commandent le basculement du mode AAI vers le mode DDD lorsqu'est vérifiée l'une au moins des conditions suivantes :
1°) le nombre d'événements auriculaires consécutifs non suivis d'une détection ventriculaire excède une première valeur autorisée, de préférence deux événements ;
2°) le nombre d'événements auriculaires non suivis d'une détection ventriculaire excède une deuxième valeur autorisée, de préférence trois événements, sur la durée d'une fenêtre de surveillance s'étendant sur un troisième nombre donné d'événements auriculaires, de préférence douze événements ;
3°) le nombre d'événements auriculaires suivis d'une détection ventriculaire survenant après un délai supérieur à une durée prédéterminée excède une quatrième valeur autorisée, de préférence six événements pour une durée prédéterminée d'au moins 300 ms en cas de détection auriculaire, ou d'au moins 350 ms en cas de stimulation auriculaire ;
4°) l'intervalle séparant deux événements ventriculaires excède un délai prédéterminé autorisé, de préférence trois secondes,
les extrasystoles auriculaires éventuelles n'étant pas comptabilisées comme des événements auriculaires.

Inversement, les moyens de commutation de mode commandent le basculement du mode DDD vers le mode AAI lorsqu'est détecté le retour d'une activité ventriculaire spontanée sur un nombre de cycles consécutifs excédant une cinquième valeur donnée, de préférence douze cycles, ou bien après un nombre d'événements ventriculaires excédant une sixième valeur autorisée, de préférence cent événements.

Avantageusement, les moyens de commutation de mode inhibent tout basculement de mode et forcent le mode de fonctionnement au mode DDD lorsque, sur un premier laps de temps prédéterminé, le nombre de commutations de AAI vers DDD excède une septième valeur prédéterminée, de préférence quinze commutations pour 24 heures, ou bien lorsque, sur un deuxième laps de temps prédéterminé, la fréquence de ces commutations excède une huitième valeur prédéterminée, de préférence cinq commutations par jour, sur trois jours.

Le dispositif peut également comprendre des moyens de détection d'extrasystoles ventriculaires, et des moyens pour délivrer une stimulation auriculaire synchrone en cas d'extrasystole ventriculaire détectée, indépendamment du mode de fonctionnement, AAI ou DDD.

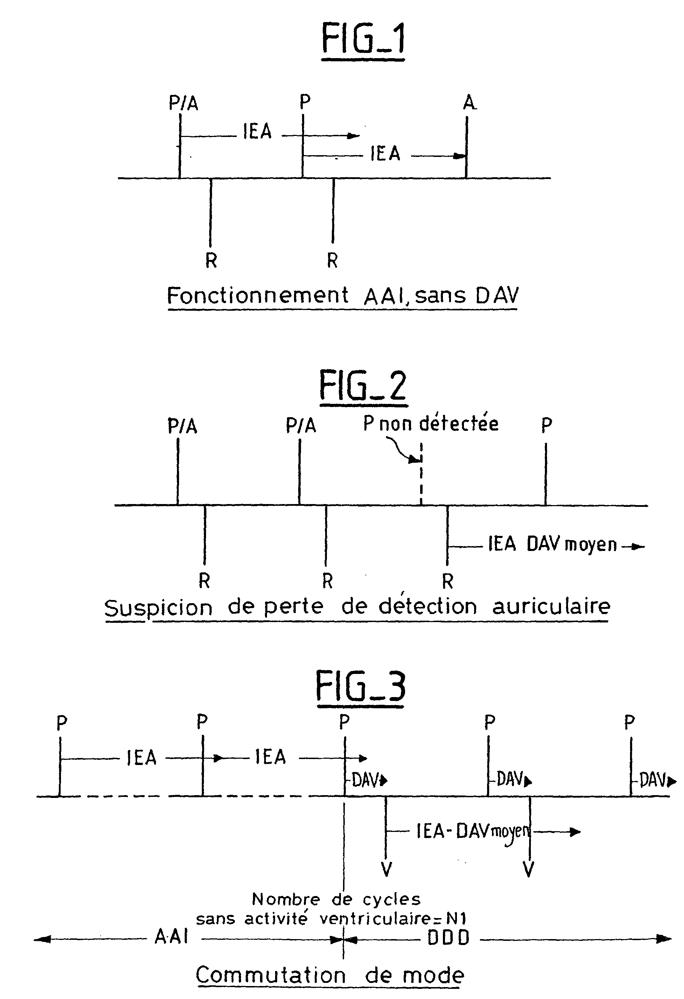

On va maintenant décrire un exemple de mise en oeuvre de l'invention, en référence aux figures annexées, où les figures 1 à 5 sont des chronogrammes correspondant aux divers modes de fonctionnement du dispositif de l'invention.

L'invention peut être mise en oeuvre par une programmation appropriée du logiciel de commande d'un stimulateur connu de type double chambre intégrant un mode DDD et un mode AAI avec surveillance de l'activité ventriculaire.

On va tout d'abord donner un certain nombre de définitions utilisées dans la suite de la description.
*Détection P* : recueil d'une activité spontanée ayant son origine dans l'oreillette ; on considérera qu'il y a effectivement détection P si celle-ci n'est pas suivie dans un délai donné, par exemple dans les 31 ms, par une détection ventriculaire (sinon, on se trouverait dans une situation de "*far-field* ventriculaire", c'est-à-dire de recueil via l'oreillette d'une dépolarisation lointaine provenant du ventricule).
*Détection R* : recueil d'une activité spontanée ayant son origine dans le ventricule.
*Stimulation A* : stimulation délivrée à l'oreillette.
*Stimulation V* : stimulation délivrée au ventricule.
*Événement auriculaire* : détection P ou bien stimulation A ;
*Événement ventriculaire* : détection R ou bien stimulation V ;
*Cycle cardiaque* : intervalle de temps séparant deux événements de même nature dans la même cavité, par exemple séparant deux détections P, ou deux stimulations A.
*PP moyen* : intervalle moyen du rythme auriculaire, calculé par exemple sur huit cycles cardiaques ne comprenant pas d'extrasystole.
*Intervalle d'échappement (IE)* : intervalle de temps, compté après une détection ou une stimulation dans une cavité donnée, à l'issue duquel une stimulation est délivrée à cette cavité si aucun événement spontané n'a été détecté dans cette même cavité. Pour l'oreillette, il s'agit de l'intervalle d'échappement auriculaire (IEA).
*Extrasystole auriculaire (ESA)* : il s'agit d'une détection auriculaire survenant à l'intérieur de la période réfractaire auriculaire post-auriculaire (PRAPA), le calcul de cette PRAPA étant celui d'un stimulateur de type DDD standard.
*Extrasystole ventriculaire (ESV)* : une détection ventriculaire est une ESV lorsqu'elle est précédée d'une détection ou d'une stimulation ventriculaire, et lorsque l'intervalle de couplage (intervalle R-R ou intervalle V-R) est inférieur ou égal à une valeur paramétrable, par exemple 75 %, du PP moyen.

Pour de plus amples détails sur la détection et le traitement des extrasystoles, on pourra se référer au EP-A-0 550 342 (Ela Médical), qui décrit un algorithme de détection et de traitement des ESV par une stimulation asynchrone de l'oreillette et une stimulation contrôlée du ventricule.

Pour la mise en oeuvre de l'invention, un certain nombre de fonctions, si elles sont présentes, sont maintenues telles quelles : ainsi, les algorithmes de stimulation, de repli (*fallback*) et de prévention des tachycardies réentrantes électroniques (TRE ou PMT, *Pacemaker-Mediated Tachycardia*) sont maintenus, de même que ceux permettant de calculer et d'appliquer des périodes PRAPA et de protection contre une conduction rétrograde en cas de suspicion d'ESV.

On va maintenant exposer la manière dont est mise en oeuvre l'invention.

Au départ, le fonctionnement du stimulateur est un fonctionnement en mode AAI avec surveillance de l'activité ventriculaire (chronogramme de la figure 1), c'est-à-dire qu'une détection auriculaire (détection P) ou une stimulation auriculaire (stimulation A) ne démarrent pas de DAV, mais démarrent un intervalle d'échappement auriculaire IEA.

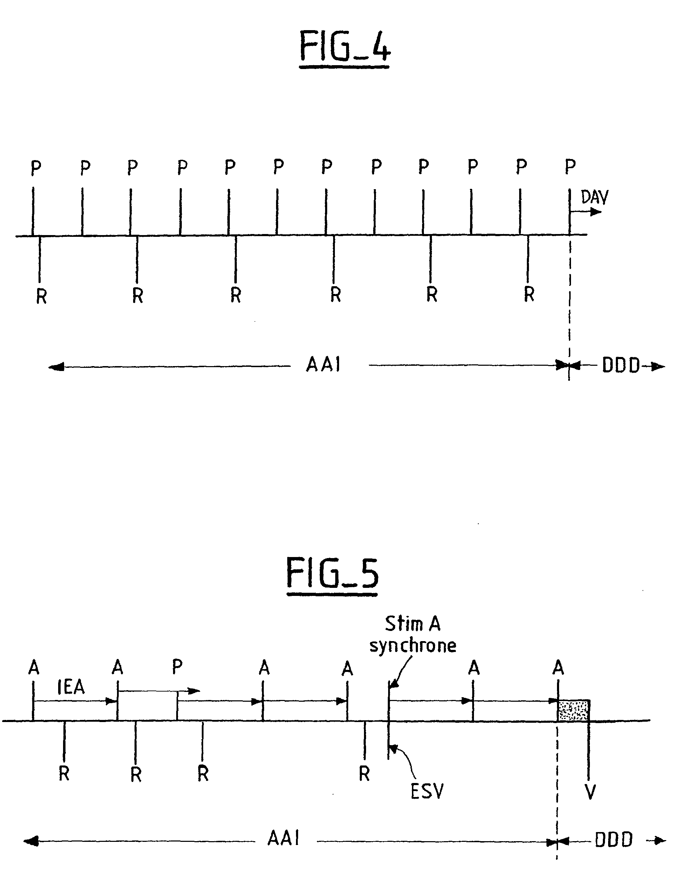

Dans ce mode AAI, l'absence d'activité ventriculaire est acceptée sur un nombre donné N1 de cycles sans déclencher de stimulation ventriculaire. Ce nombre N1 est programmable, par exemple N1 = 2 cycles (cf. notamment la situation illustrée sur la partie gauche du chronogramme de la figure 3).

En cas de détection ventriculaire, cette détection n'a normalement pas d'effet sur l'IEA, sauf en cas de suspicion de perte de détection auriculaire.

Ce cas de suspicion de perte de détection auriculaire est illustré par le chronogramme de la figure 2.

La situation est celle d'une détection ventriculaire R non précédée d'une détection auriculaire P, et en l'absence d'accélération du rythme - une telle accélération étant détectée lorsque l'intervalle de couplage (intervalle RR) franchit un seuil prédéterminé. Dans ce cas, l'algorithme suspecte non pas une ESV (car dans ce cas il y aurait eu accélération du rythme), mais un défaut de détection auriculaire, et relance un intervalle d'échappement auriculaire de valeur égale à (IEA - DAV moyen).

Le mode AAI étant pourvu d'une surveillance de l'activité ventriculaire, l'algorithme recherche par ailleurs la présence ou l'absence d'une activité ventriculaire, qui dans ce dernier cas pourrait laisser suspecter un BAV, de manière à éventuellement basculer en mode DDD de stimulation double chambre avec association atrio-ventriculaire, c'est-à-dire avec calcul et application d'un DAV.

Cette situation de basculement de mode correspond au chronogramme de la figure 3.

Quatre critères peuvent déclencher la commutation de mode de AAI vers DDD:
1°) le nombre d'événements auriculaires bloqués (c'est-à-dire non suivis d'une détection ventriculaire) consécutifs excède la valeur programmable autorisée N1 ci-dessus (par exemple N1 = 2) ; ou
2°) le nombre d'événements auriculaires bloqués excède une valeur programmable N2 autorisée (par exemple N2 = 3) sur une fenêtre de surveillance de N3 événements auriculaires, N3 étant un nombre programmable, par exemple N3 = 12 événements - cette situation est illustrée notamment sur le chronogramme de la figure 4 ; ou
3°) le nombre d'événements auriculaires suivis d'une détection ventriculaire après un délai anormalement long (délai paramétrable, par exemple 300 ms) excède une valeur programmable autorisée N4, par exemple N4 = 6 événements ; ou
4°) la pause ventriculaire (c'est-à-dire l'intervalle séparant deux événements ventriculaires) excède le délai programmable autorisé, par exemple 3 secondes.

En présence de l'un de ces critères, le dispositif bascule du mode AAI en mode DDD avec DAV. Les valeurs de DAV sont soit celles initialement programmées par le praticien, soit celles calculées automatiquement par l'algorithme (on pourra se référer à cet égard au EP-A-0 488 904 précité).

Le DAV est activé sur tout événement auriculaire qui n'est pas une ESA, et ce jusqu'au retour d'une activité ventriculaire spontanée sur un nombre programmable N5 de cycles consécutifs, par exemple N5 = 12 cycles, ou bien après un nombre maximal programmable N6 d'événements ventriculaires, par exemple N6 = 100 événements.

Le dispositif commute alors de DDD en AAI et demeure en mode AAI tant qu'aucun des quatre critères précités de commutation de AAI vers DDD n'est vérifié.

Il est souhaitable de prévoir une limitation au nombre de commutations successives de AAI vers DDD sur une période donnée.

Ainsi, par exemple :
- si, sur une période de 24 h, le nombre de commutations excède un nombre maximal programmable N7 de fois, par exemple N7 = 15 fois, ou
- si, pendant trois jours consécutifs, le nombre quotidien de commutations excède un nombre maximal programmable N8 de fois, par exemple N8 = 5 fois, par 24 h,
alors le dispositif bascule définitivement en mode DDD, avec les valeurs de DAV programmées à l'expédition de l'appareil, ou bien celles programmées à l'implantation, et ce jusqu'à reprogrammation ultérieure par le praticien.

L'algorithme gère également les ESV : en cas d'ESV détectée, une stimulation auriculaire synchrone est délivrée par le dispositif, que le stimulateur soit en mode DDD ou en mode AAI. Cette dernière situation est notamment illustrée sur le chronogramme de la figure 5.

## Revendications

1. Un dispositif médical implantable actif, notamment un stimulateur cardiaque, défibrillateur et/ou cardioverteur, comprenant :
- des moyens de détection d'événements auriculaires et ventriculaires spontanés,
- des moyens de stimulation ventriculaire et auriculaire,
- des moyens aptes à faire fonctionner le dispositif en mode DDD,
- des moyens aptes à faire fonctionner le dispositif en mode AAI avec détection ventriculaire, et
- des moyens de commutation de mode, aptes à commander le basculement du mode AAI en mode DDD, et inversement, en fonction de critères prédéterminés,
dispositif **caractérisé en ce que** lesdits moyens de commutation de mode commandent le basculement du mode AAI vers le mode DDD lorsqu'est vérifiée l'une au moins des conditions suivantes :
1°) le nombre d'événements auriculaires consécutifs non suivis d'une détection ventriculaire excède une première valeur autorisée (N1) ;
2°) le nombre d'événements auriculaires non suivis d'une détection ventriculaire excède une deuxième valeur autorisée (N2) sur la durée d'une fenêtre de surveillance s'étendant sur un troisième nombre donné (N3) d'événements auriculaires ;
3°) le nombre d'événements auriculaires suivis d'une détection ventriculaire survenant après un délai supérieur à une durée prédéterminée excède une quatrième valeur autorisée (N4) ;
4°) l'intervalle séparant deux événements ventriculaires excède un délai prédéterminé autorisé,
les extrasystoles auriculaires éventuelles n'étant pas comptabilisées comme des événements auriculaires.

2. Le dispositif de la revendication 1, dans lequel ladite première valeur (N1) est de deux événements.

3. Le dispositif de la revendication 1, dans lequel ladite deuxième valeur (N2) est de trois événements pour une troisième valeur (N3) de douze événements.

4. Le dispositif de la revendication 1, dans lequel ladite quatrième valeur (N4) est de six événements, pour une durée prédéterminée séparant événements auriculaires et détections ventriculaires consécutives d'au moins 300 ms en cas de détection auriculaire, ou d'au moins 350 ms en cas de stimulation auriculaire.

5. Le dispositif de la revendication 1, dans ledit délai prédéterminé autorisé séparant deux événements ventriculaires est de trois secondes.

6. Le dispositif de la revendication 1, dans lequel lesdits moyens de commutation de mode commandent le basculement inverse du mode DDD vers le mode AAI lorsqu'est détecté le retour d'une activité ventriculaire spontanée sur un nombre de cycles consécutifs excédant une cinquième valeur donnée (N5).

7. Le dispositif de la revendication 6, dans lequel ladite cinquième valeur (N5) est de douze cycles.

8. Le dispositif de la revendication 1, dans lequel lesdits moyens de commutation de mode commandent le basculement inverse du mode DDD vers le mode AAI après un nombre d'événements ventriculaires excédant une sixième valeur autorisée (N6).

9. Le dispositif de la revendication 8, dans lequel ladite sixième valeur (N6) est de cent événements.

10. Le dispositif de la revendication 1, dans lequel lesdits moyens de commutation de mode inhibent tout basculement de mode et forcent le mode de fonctionnement au mode DDD lorsque, sur un premier laps de temps prédéterminé, le nombre de commutations de AAI vers DDD excède une septième valeur prédéterminée (N7).

11. Le dispositif de la revendication 10, dans lequel ladite septième valeur (N7) est de quinze commutations, pour un premier laps de temps de 24 heures.

12. Le dispositif de la revendication 1, dans lequel lesdits moyens de commutation de mode inhibent tout basculement de mode et forcent le mode de fonctionnement au mode DDD lorsque, sur un deuxième laps de temps prédéterminé, la fréquence des commutations de AAI vers DDD excède une huitième valeur prédéterminée (N8).

13. Le dispositif de la revendication 12, dans lequel ladite huitième valeur (N8) est de cinq commutations par jour, pour un deuxième laps de temps de trois jours.

14. Le dispositif de la revendication 1, comprenant en outre des moyens de détection d'extrasystoles ventriculaires, et des moyens pour délivrer une stimulation auriculaire synchrone en cas d'extrasystole ventriculaire détectée, indépendamment du mode de fonctionnement, AAI ou DDD.

## Patentansprüche

1. Aktive implantierbare medizinische Vorrichtung, insbesondere ein Herzschrittmacher, Defibrillator und/oder Cardioverter, aufweisend:
- Mittel zur Detektion von spontanen atrialen und ventrikulären Ereignissen,
- Mittel zur atrialen und ventrikulären Stimulation,
- Mittel, angepasst, die Vorrichtung in einem DDD-Modus arbeiten zu lassen,
- Mittel, angepasst, die Vorrichtung in einem AAI-Modus mit ventrikulärer Detektion arbeiten zu lassen, und
- Mittel zur Umschaltung des Modus, angepasst, das Umschalten des AAI-Modus in den DDD-Modus und umgekehrt zu veranlassen, in Abhängigkeit von vorbestimmten Kriterien,
wobei die Vorrichtung **dadurch gekennzeichnet ist, dass** die Mittel zum Umschalten des Modus das Umschalten des AAI-Modus in den DDD-Modus veranlassen, wenn mindestens eine der folgenden Bedingungen erfüllt ist:
1. die Anzahl der aufeinander folgenden atrialen Ereignisse, welche nicht von einer ventrikulären Detektion gefolgt werden, überschreitet einen ersten erlaubten Wert (N1);
2. die Anzahl der atrialen Ereignisse, die nicht von einer ventrikulären Detektion gefolgt werden, überschreitet einen zweiten erlaubten Wert (N2) über die Dauer eines Beobachtungsfensters, welches sich über eine dritte gegebene Anzahl (N3) von atrialen Ereignissen erstreckt;
3. die Anzahl der atrialen Ereignisse, denen eine ventrikuläre Detektion folgt, die nach einer Verzögerung auftritt, welche höher ist als eine vorbestimmte Dauer, überschreitet einen vierten erlaubten Wert (N4);
4. das Intervall, das zwei aufeinander folgende ventrikuläre Ereignisse trennt, überschreitet eine vorgegebene erlaubte Verzögerung, wobei die eventuellen atrialen Extrasystolen nicht als atriale Ereignisse berücksichtigt werden.

2. Vorrichtung nach Anspruch 1, bei welcher der erste Wert (N1) zwei Ereignisse ist.

3. Vorrichtung nach Anspruch 1, bei welcher der zweite Wert (N2)drei Ereignisse ist, für einen dritten Wert (N3) von zwölf Ereignissen.

4. Vorrichtung nach Anspruch 1, bei welcher der vierte Wert (N4) sechs Ereignisse ist, für eine vorbestimmte Dauer, welche atriale Ereignisse und ventrikuläre Detektionen trennt, die wenigstens 300 ms aufeinander folgen, im Fall einer atrialen Detektion, oder wenigstens 350 ms, im Fall einer atrialen Stimulation.

5. Vorrichtung nach Anspruch 1, bei der die vorbestimmte maßgebliche Verzögerung, welche zwei ventrikuläre Ereignisse trennt, drei Sekunden beträgt.

6. Vorrichtung nach Anspruch 1, bei der die Umschaltmittel das umgekehrte Umschalten vom DDD-Modus in den AAI-Modus veranlassen, wenn die Rückkehr einer ventrikulären spontanen Aktivität über eine Anzahl von aufeinander folgenden Zyklen festgestellt wird, die einen fünften gegebenen Wert (N5) überschreitet.

7. Vorrichtung nach Anspruch 6, bei welcher der fünfte Wert (N5) zwölf Zyklen ist.

8. Vorrichtung nach Anspruch 1, bei welcher die Mittel zur Umschaltung des Modus die Umschaltung vom DDD-Modus zurück in den AAI-Modus veranlassen, nach einer Anzahl von ventrikulären Ereignissen, die einen sechsten zulässigen Wert (N6) überschreitet.

9. Vorrichtung nach Anspruch 8, bei welcher der sechste Wert (N6) hundert Ereignisse beträgt.

10. Vorrichtung nach Anspruch 1, bei welcher die Modusumschaltmittel jegliche Umschaltung des Modus verhindern und den Betriebsmodus im DDD-Modus erzwingen, wenn nach dem Verstreichen einer vorbestimmten Zeit die Anzahl des Umschaltens von AAI nach DDD einen siebten vorbestimmten Wert (N7) übersteigt.

11. Vorrichtung nach Anspruch 10, bei welcher der siebte Wert (N7) fünfzehn Umschaltvorgänge beträgt, für einen ersten verstrichenen Zeitraum von 24 Stunden.

12. Vorrichtung nach Anspruch 1, bei der die Mittel zur Umschaltung des Modus jegliches Umschalten des Modus verhindern und den Betriebsmodus im DDD-Modus erzwingen, wenn in einer vorbestimmten zweiten Zeitspanne die Frequenz der Umschaltungen von AAI nach DDD einen achten vorbestimmten Wert (N8) übersteigt.

13. Vorrichtung nach Anspruch 12, bei welcher der achte Wert (N8) fünf Umschaltungen pro Tag beträgt, für eine zweite Zeitspanne von drei Tagen.

14. Vorrichtung nach Anspruch 1, die außerdem Mittel zur Detektion von ventrikulären Extrasystolen aufweist und Mittel zur Abgabe einer synchronen atrialen Stimulation im Fall einer detektierten ventrikulären Extrasystole, unabhängig vom Betriebsmodus AAI oder DDD.

## Claims

1. An active implantable medical device, in particular a cardiac pacemaker, defibrillator and/or cardioverter, comprising:
- means for detection of spontaneous atrial and ventricular events,
- means for ventricular and atrial stimulation,
- means adapted to operate the device in DDD mode,
- means adapted to operate the device in AAI mode with ventricular detection, and
- mode commuting means, adapted to control switching from AAI mode to DDD mode, and vice versa, as a function of predetermined criteria,
said device being **characterised in that** said mode commuting means control switching from AAI mode to DDD mode when at least one of the following conditions is met:
1) the number of consecutive atrial events which are not followed by a ventricular detection is greater than a first permitted value (N1);
2) the number of atrial events which are not followed by a ventricular detection is greater than a second permitted value (N2) for the duration of a monitoring window encompassing a third given number (N3) of atrial events;
3) the number of atrial events which are followed by a ventricular detection occurring after a delay being greater than a predetermined duration is greater than a fourth permitted value (N4);
4) the interval separating two ventricular events is greater than a predetermined permitted duration,
the possible atrial extrasystote being not counted as atrial events.

2. The device of claim 1, wherein said first value (N1) is two events.

3. The device of claim 1 wherein said second value (N2) is three events for a third value (N3) being twelve events.

4. The device of claim 1, wherein said fourth value (N4) is six events, for a predetermined duration separating atrial events and consecutive ventricular detections of at least 300 ms in case of atrial detection, or at least 350 ms in cases of atrial stimulation.

5. The device of claim 1, wherein said predetermined permitted duration separating two ventricular events is three seconds.

6. The device of claim 1 wherein said mode commuting means controls reverse switching from DDD mode to AAI mode when a return is detected to a spontaneous ventricular activity over a number of consecutive cycles greater than a fifth given value (N5).

7. The device of claim 6 wherein said fifth value (N5) is twelve cycles.

8. The device of claim 1, wherein said mode commuting means controls reverse switching from DDD mode to AAI mode after a number of ventricular events greater than a sixth permitted value (N6).

9. The device of claim 8, wherein said sixth value (N6) is one hundred events.

10. The device of claim 1, wherein said mode commuting means inhibits any mode switching and forces the operating mode to the DDD mode when, over a first predetermined time span, the number of commutations from AAI to DDD is greater than a seventh predetermined value (N7).

11. The device of claim 10, wherein said seventh value (N7) is fifteen commutations, for a first time span of 24 hours.

12. The device of claim 1, wherein said mode commuting means inhibits any mode switching and forces the operating mode to the DDD mode when, over a second predetermined time span, the rate of commutations from AAI to DDD is greater than a eight predetermined value (N8).

13. The device of claim 12, wherein said eighth value (N8) is five commutations per day, for a second time span of three days.

14. The device of claim 1, further comprising means for detecting ventricular extrasystoles, and means for delivering a synchronous atrial stimulation in case of a detected ventricular extrasystole, whatever be the operating mode, AAI or DDD.
